(19) Europäisches Patentamt / European Patent Office / Office européen des brevets

(11) **EP 4 486 439 B1**

(12) **EUROPEAN PATENT SPECIFICATION**

(45) Date of publication and mention
of the grant of the patent:
**19.11.2025  Bulletin 2025/47**

(21) Application number: **23707981.9**

(22) Date of filing: **03.03.2023**

(51) International Patent Classification (IPC):
**A61N 5/06** (2006.01)         **A61N 5/10** (2006.01)

(52) Cooperative Patent Classification (CPC):
**A61N 5/0625;** A61N 5/1002; A61N 5/1014

(86) International application number:
**PCT/EP2023/055488**

(87) International publication number:
**WO 2023/166202 (07.09.2023 Gazette 2023/36)**

(54) **SYSTEM FOR RADIATION WITH HYPERTHERMIA TREATMENT**

SYSTEM ZUR BESTRAHLUNG MIT HYPERTHERMIEBEHANDLUNG

SYSTÈME DE RAYONNEMENT AVEC TRAITEMENT PAR HYPERTHERMIE

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **04.03.2022  EP 22160317**

(43) Date of publication of application:
**08.01.2025  Bulletin 2025/02**

(73) Proprietors:
• **Les Hôpitaux Universitaires de Genève**
**1205 Geneva (CH)**
• **UNIVERSITE DE GENEVE**
**1211 Geneva 4 (CH)**

(72) Inventors:
• **DIPASQUALE, Giovanna**
**1255 Veyrier (CH)**

• **UITERWIJK, Johannes**
**1255 Veyrier (CH)**
• **GUILLEMIN, Pauline**
**74100 Annemasse (FR)**
• **SALOMIR, Rares**
**01200 Valserhône (FR)**
• **LORTON, Orane**
**74100 Ambilly (FR)**

(74) Representative: **reuteler & cie SA**
**Chemin de la Vuarpillière 29**
**1260 Nyon (CH)**

(56) References cited:
**US-A- 5 613 940        US-A1- 2011 040 190
US-A1- 2019 365 462        US-A1- 2020 060 356
US-A1- 2020 093 374**

EP 4 486 439 B1

## Description

### Field of the invention

This invention relates to a system for radiation with hyperthermia treatment of cancer.

### Background of the invention

External beam radiotherapy (EBRT) is the gold standard salvage treatment for patients experiencing a biochemical relapse after radical prostatectomy (RP) [1]. Although it has been improved for selected patients by combining androgen deprivation [2, 3], the long-term disease control remains limited, with approximately 50% of the patients presenting a disease progression at five years [4]. In analogy with clinical prospective phase III trials of radical EBRT for localized prostate cancer, dose escalation is used to improve outcome even in the setting of salvage radiotherapy, although long-term toxicities may be increased especially when the radiotherapy doses exceed 70 Gy [5-8].

More compelling treatment approaches able to improve the therapeutic ratio in the salvage setting are therefore needed, especially for patients harboring a macroscopic relapse in the prostate bed [5]. Hyperthermia (HT) [9] with radiation is a promising treatment modality relying on well-established rationale [10] and clinical data [11-13]. By increasing the temperature of the targeted region to values between 41° and 43°, deep HT combined with EBRT could improve the cell killing [14-16] and potentially reduce side-effects by avoiding the need of dose escalation or androgen deprivation.

With its capacity to sharply deposit energy inside deep tissues, magnetic resonance-guided focused ultrasound technology (MRgFUS) employing modern phased array transducers has been proposed as a promising technology for deep HT [17, 18]. In the last decade the number of potential applications of this technology has rapidly increased with multiple studies proving its application in different tumor sites for ablative [19-25] or HT purposes [26-29].

In order to spare surrounding healthy tissues, use of image-guided radiation therapy (IGRT) and positioning devices help to improve precise coverage of the target volume during radiation therapy for cancer treatment. A correct daily repositioning has been associated with a better long-term disease control and a reduced rate of radiation-induced toxicities for treatment of prostate cancer [30]. Tattoo is a commonly used method to mark skin to line up the radiotherapy machine for each treatment. However, radiotherapy tattoos can be a constant reminder of disease and tattoo removal is commonly not covered by health insurance [31]. In addition, delivering adjuvant HT with MRgFUS requires patient immobilization and accurate positioning of the applicator, which should be reproducible during a multiple sessions treatment.

High-precision targeting is required for both EBRT and ultrasound HT techniques to avoid overtreatments in the surrounding normal tissues [30].

US 2011/040190 teaches a system being devoid of a treatment process computing system as set out in present claim 1.

### Summary of the invention

In view of the foregoing, it is an object of this invention to provide a system for radiation with hyperthermia treatment of cancer that allows to accurately target the zone to be treated in a safe and efficient reproducible manner over a plurality of radiation treatment cycles.

It is advantageous to provide a system for radiation with hyperthermia treatment that is economical to implement.

It is advantageous to provide a system for radiation with hyperthermia treatment that reduces discomfort to the patient.

It is advantageous to provide a system for radiation with hyperthermia treatment that reduces the treatment time and patient immobilization time.

It is advantageous to provide a system for radiation with hyperthermia treatment that reduces the complexity of treatment setup for health care practiners.

Objects of this invention have been achieved by providing a system for radiation with hyperthermia treatment according to claim 1.

Dependent claims set forth various advantageous features and embodiments of the invention.

Disclosed herein is a system for radiation with hyperthermia cancer treatment comprising a rigid support shell, the support shell comprising a body conforming wall and at least one treatment device locator, the treatment device locator comprising an orifice and a guide tube upstanding outwardly from the body conforming wall and configured to receive and locate a treatment device including a hyperthermia applicator.

According to a first aspect, the support shell comprises at least two separable parts removably fixable together for positioning around a body part of a patient.

According to a second aspect, the treatment device locator and the treatment device optionally with device adaptor couple together define a specific position on the support shell and a specific target therapy direction (*La*) relative to the support shell, and the treatment device locator and treatment device, optionally with device adaptor, interface with a pre-defined amount of play allowing a specified degree of freedom in the direction of the treatment device relative to the axis of treatment (*La*), the adjustment being within a cone having an angle of less than 10°, preferably less than 5°.

According to a third aspect, the system comprises a treatment process computing system including a program module configured for controlling a treatment process in which data of a first session of HT under MRI control is recorded in the computing system, the program module comprising a Green function computation module configured to compute a prescribed power over time, based on said recorded data of a first session and a target tissue temperature, for outputting process control data for controlling the ultrasound hyperthermia applicator device in subsequent sessions without MRI control.

In an advantageous embodiment the system further comprises a device adaptor having an adjustable or tunable interface coupling to the guide tube of the treatment device locator and an inner interface locating a treatment device.

In an advantageous embodiment the system further comprises said treatment device.

The treatment device may include any one or more of a hyperthermia applicator, a radiation emitter and a monitoring device. In an advantageous embodiment the treatment device includes the hyperthermia applicator.

In an advantageous embodiment the hyperthermia applicator is a hyperthermia multi-channel applicator.

In an advantageous embodiment the support wall consists of or is principally made of a polymer material, preferably selected from a group consisting of Acrylonitrile Butadiene Styrene, Polylactic Acid and Nylon.

In an advantageous embodiment the support wall has an average thickness in a range of 1-7mm, preferably in a range of 3-7mm.

The system may further comprise a computing system and image processing software installed therein configured to generate a 3-dimensional form of the support shell and to further generate a manufacturing file from the 3-D form of the support shell for transmission to a support shell manufacturing machine.

In an advantageous embodiment the system further comprises a support shell manufacturing machine including an additive manufacturing device.

In an advantageous embodiment the additive manufacturing device is a 3-D printing device.

In an advantageous embodiment the guide tube is cylindrical. In variants, the guide tube may however have other shapes such as elliptical, square, rectangular, polygonal and various irregular shapes.

Also disclosed herein is a method of manufacturing a support shell for radiation with hyperthermia treatment comprising : processing in a computer system a scan file obtained from a scan of a body part and a treatment device applied to the body part, generating a support shell shape including a locator on the support shell at a position surrounding a position of the treatment device in the scan file, generating a manufacturing file from the shape file, transmitting the manufacturing file to a manufacturing machine and manufacturing the support shell.

Further objects and advantageous aspects of the invention will be apparent from the claims, and from the following detailed description and accompanying figures.

**Brief description of the drawings**

**Figures 1A a to f** are different perspective views of a support shell of a system for radiation with hyperthermia cancer treatment according to embodiments of the invention, whereby figures 1a to 1c illustrate views of a first body shell, and figures 1d to 1f show views of a second body shell, figure 1f showing one half of the body shell;

**Figures 1B a to f** are photographic views of manufactured samples of the support shell of figure 1A;

**Figures 2a to 2f** illustrate different photographic views of a support shell in experimental use;

**Figure 3A a and b** illustrate use of a treatment or imaging ultrasound device and adaptor therefor of a system for radiation with hyperthermia treatment according to an embodiment of the invention;

**Figure 3B a and b** are photographic views of manufactured samples of the imaging ultrasound device and adaptor therefor of figure 3A;

**Figures 4a to 4c** illustrate a 3-dimensional computer model of a support shell to be manufactured;

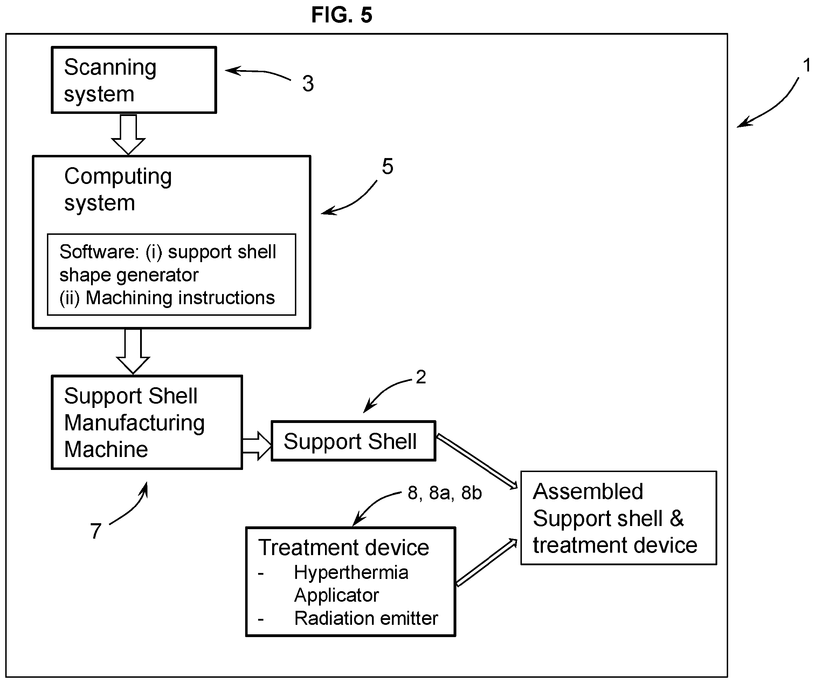

**Figure 5** illustrates a simplified block diagram of a system for radiation with hyperthermia treatment method for treatment of a patient according to embodiments of the invention;

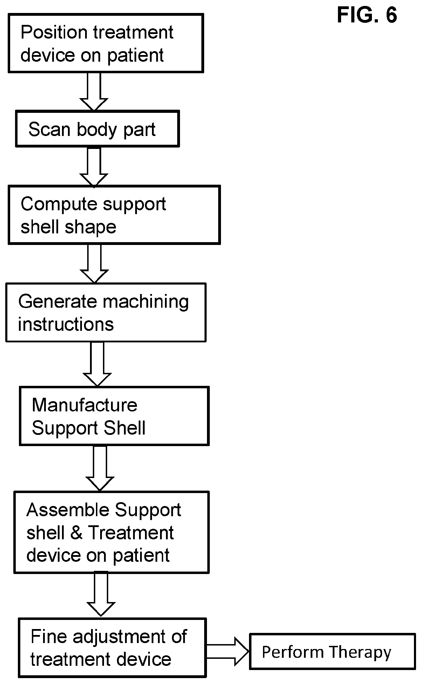

**Figure 6** is a schematic flow diagram illustrating a method of preparing a system for radiation with hyperthermia treatment according to an embodiment of the invention;

**Figure 7** illustrates a simplified block diagram of a treatment process of a system for radiation with hyperthermia treatment according to embodiments of the invention;

**Figure 8** illustrate plots of a measured power driving an ultrasound hyperthermia applicator and resulting measured tissue temperature over time, and using a Green function, the computed plot of a prescribed power based on a target tissue temperature, for a system for radiation with hyperthermia treatment according to an embodiment of the invention.

## Detailed description of embodiments of the invention

Referring to the figures, a system for radiation with hyperthermia cancer treatment 1 comprises a scanning system 3, a computing system 5, a support shell manufacturing machine 7, one or more treatment devices 8, and a support shell 2 manufactured by the support shell manufacturing machine using data collected by the scanning system 3 and processed by the computing system 5. The scanning system may for instance comprise a patient support table, transparent elements and a surface scanner, as *per se* known.

The support shell 2 forms a personalized immobilisation device comprising a body conforming wall 4 on which one or more treatment device locators 6 are integrated. In the illustrated example, the support shell conforms to the pelvis of a patient and is configured for prostate cancer treatment using radiation therapy with hyperthermia.

Within the scope of the invention, the support shell may conform to other body parts for other cancer treatments. For instance, the support shell may have a shape configured to conform to a breast for breast cancer treatment, a waist for cancer treatment of various internal organs such as liver, bladder, stomach and other cancer treatments, a neck for a treatment of throat cancer, a head for treatment of brain cancer, and various other body parts including around limbs.

The treatment device locator 6 is configured to receive and locate a treatment device 8 including a hyperthermia applicator 8a and a radiation emitter 8b and optionally monitoring devices. The hyperthermia applicator may be a hyperthermia single channel or multi-channel applicator. Multi-channel applicators advantageously allow electronically controlled stirring of the heating. The treatment device 8 may further comprise a device adaptor 10 fitting around or mounted to the specific treatment device, providing an interface between the shape of the treatment device and the shape of the treatment device locator 6 on the support shell 2. This allows different treatment devices, for instance the hyperthermia applicator and the radiation emitter to be positioned by the treatment device locator 6 on the support shell 2 despite the different outer shapes of the respective treatment devices. The coupling adaptor may thus be used to form an interface between the support shell treatment device locator 6 and the treatment device and various treatment devices. Moreover, treatment can be performed with different devices having different shapes that are adapted to the locator 6 by the device adaptor 10. The device adaptor may advantageously provide an interface that also allows the treatment device to be coupled to the support shell by fixing means such as clamps, screws, clips and other fixing means (not shown) provided on the device adaptor configured to couple to complementary means on the treatment device locator 6 on the support shell 2 in order to securely fix the treatment device to the support shell during treatment.

The device adaptor and treatment device locator 6 may further comprise an interface that allows certain positional adjustment, for instance allowing a certain angle of inclination with respect to the locator center axis *La* in order to adjust the position of the treatment device with respect to the support shell.

In an advantageous embodiment, the treatment device locator 6 comprises an orifice 14 in the body conforming wall 4 of the support shell at a selected treatment position, and a guide tube 14 or rim having an outer edge 16 upstanding outwardly from the body conforming wall 4. The guide tube 14 is configured to locate therein a front end of the device adaptor 10, or a front end of the treatment device 8 in case no device adaptor is used, and provides a direction *La* for the treatment device as well as a height positioning with respect to the body conforming wall 4.

As mentioned, the guide tube relative to the device adaptor respectively treatment device may be provided with a certain amount of play or positional tolerance allowing a certain inclination about the center axis of the radiation focus direction in order to provide a fine adjustment of the treatment device focussing direction during treatment.

The support shell may advantageously be manufactured from a rigid thermo-plastic or thermo-setting polymer, preferably by additive manufacturing such as using 3-D printing techniques. Other manufacturing processes however may also be used such as subtractive manufacturing processes using laser cutting or ablation or machine tool milling, turning and drilling operations. Subtractive manufacturing techniques may advantageously be used to prepare the guide tube 14 inner surface and other locating surfaces of the treatment device locator 6 on the support shell 2.

The support shell may be provided advantageously in two parts for mounting around a body portion of the person subject to therapy. The two body parts preferably comprise locator elements such as a protuberance and corresponding indent on the shell halves that inter-engage when mounted around the patient body portion for accurate location and seating thereof. The shell fixing means such as a belt, strap, clips, clamps, screws and other *per se* known fixing means may be used to clamp the body shells together when in use for treatment.

The device adaptor 10 may comprise a container shaped wall into which the corresponding treatment device, such as the hyperthermia applicator or the radiation emitter is inserted. A front end of the device adaptor comprises an orifice to allow the treatment device front end to not be covered by the adaptor and for instance be pressed against the skin of the patient when in position for treatment.

In the illustrated embodiment, the support shell 2 is provided with a single treatment device locator 6, however in variants it is possible to provide two or more treatment device locators 6 of the same or of different shapes and sizes, for receiving multiple emission or monitoring devices such as an ultra-sound emission device, a radiation therapy emission device, and measurement devices such as ultra-sound measurement probes, X-ray probes, temperature sensing probes, radiation detectors and other sensing probes that may be used simultaneously during therapy with the treatment device or at different times but using a different position and angle.

In an example of treatment within the scope of the invention, the hyperthermia applicator 8a is coupled to the treatment device locator 6 and hyperthermia treatment is performed, the hyperthermia applicator may then be removed and the radiation therapy may be performed in a conventional radiation therapy machine, whereby the support shell may be used to immobilize the section of the patient's body undergoing radiation. If the radiation therapy is performed in a machine with MRI imaging, the support shell, including the device locator, may advantageously also serve as a spatial reference to locate the treatment zone.

In another example of radiation therapy with hyperthermia for cancer treatment, the hyperthermia applicator may be used in a first step and the radiation device in a second step, a pre-defined amount of time after the hyperthermia treatment using the same treatment device locator 6. The radiation emitter 8b may be placed on the treatment device locator 6 for subsequent radiation.

Multiple radiation treatments over a span of time, for instance over hours or days, may be performed, whereby the support shell 2 may be removed from the patient between treatment cycles and put back on for treatment while advantageously providing an accurate positioning due to the personalized shape of the support shell to the patient's body portion and fixed position thereon of the treatment device locator 6, thus ensuring that at each treatment cycle the treatment or monitoring devices are located in the same position and general focus angle, except for some fine adjustment that may be performed prior to each cycle of therapy.

The material of the support wall is selected to be transparent to magnetic resonance imaging devices that allow a real time tomographic scan of the tissue volume undergoing treatment. Example of materials include Acrylonitrile Butadiene Styrene ,PolyLactic Acid polymers, and Nylon.

A method of producing the support shell 2 may comprise the following steps.

First, a scan of the body part of the patient is performed to obtain a 3-D shape of the relevant body part around which the support shell is intended to be mounted. There are various *per se* known scanning machines that may be used for this process and that do not need to be further described herein. The patient may in variants advantageously wear a garment, for instance in the form of boxer shorts for prostate cancer treatment, that comprise markings that form references for the scanning procedure to improve accuracy and increase the scanning speed. A dedicated surface scanning imaging support, such as but not restricted to a table, allowing to surface scan through the "transparent elements" will permit to acquire the patients' body form deformation on the table due to gravity or other systems pressing on the body part when the patient is laying on it

The resulting scan file may be processed in the computing system 5 to generate a support shell geometric shape adapted for the manufacturing step, for instance configured for a 3-D printing step with *a per se* known 3-D printing machine.

The scanning step may advantageously be performed while a treatment device 8, in particular a hyperthermia applicator 8a, is positioned against the patient's skin and held by an external adjustable holding device (not shown) that positions the treatment device in the selected position and selected direction. The selected position and selected direction may be obtained by adjusting the position and simultaneously measuring the region of focus of tissue under treatment using a measurement device such as a magnetic resonance imaging device. The location and directional adjustment of the treatment device however may be selected by various other methods, for instance by manual selection via a skilled healthcare practioner, or by a pre-diagnosis of the patient in which the position of the tissue to be treated has been located by X-ray, ultrasound, magnetic resonance imaging or other methods and is known with respect to certain reference positions or markers on the patient's body.

After the scanning and shaping operation of the support shell, a manufacturing output file is generated for automated manufacturing via a 3-D printing or other additive or subtractive manufacturing techniques. Certain manufacturing steps may be performed independently of the manufacturing file, for instance the cutting of the support shell in two or more parts for fitting around the body part, and various features such as the clamping or other fixing means may be added to the manufacturing file. The manufacturing process may further comprise various finishing steps such as grinding, polishing, milling, drilling, including a finishing operation for the treatment device locator 6.

After the body shell is manufactured, it may then be used for therapy by placing around and fixing to the patient's body part and fixing the treatment devices to the treatment device locator 6, whereby the treatment may be performed under magnetic resonance imaging and other sensing techniques accompanying the treatment.

Hyperthermia may be performed first and within a pre-determined amount of time thereafter radiation treatment. In treatments requiring multiple therapy cycles, the support shell may be removed and at the next treatment cycle put back on the patient. Alternatively, at each treatment cycle, if they are far apart in time, a new support shell may be manufactured.

A specific experimental example of a prototype support shell for hyperthermia cancer treatment has been tested and is described below, it being understood that this is a very specific example and that the invention may be applied for various cancer treatments in various manners as described above.

## *Example*

### 1. Introduction

The personalized support shell employed in therapy ensures intra- and inter-fraction reproducibility during the whole treatment course. Three-dimensional (3D) printing is an emerging technology that can be employed to produce personalized support shell devices, able to ensure patient immobilization but mostly to integrate dedicated holders needed for hyperthermia (HT) applicators and/or organ position monitoring devices.

Standard commercially available immobilization devices such as Vac-Lok™ (CIVCO, Iowa, US), alpha cradle® (Smithers Medical Products, Ohio, US), thermoplastic masks, knee and ankle supports cannot combine this double function, as they are not suitable to be used for embedding HT applicators. Furthermore, the rigidity of a 3D-print can be used to support the weight of treatment and measurement devices such as the magnetic resonance imaging (MRI) coil weight. This avoids body deformation and helps to improve MR image quality, including MR thermometry, because of the reduced body-to-coil distance [32] and reduced motion [33].

In the context of an ongoing clinical study aiming to explore the combination of MRgFUS-based HT and salvage EBRT in patients with biochemical relapse after RP, a 3D-printed, personalized support shell adapted for EBRT and HT treatments embedding an ultrasound (US) holder was developed. Rapid and reproducible repositioning of the patient during the EBRT and HT sessions and embedding an US applicator capable of imaging a pre-defined region were the major goals of the prototype. This experimental study reports on the device characteristics and the inter-fractional set-up reproducibility on volunteers. The capabilities of the device to allow acoustic targeting of the same region of interest are evaluated, aiming for future application of therapeutic ultrasound. Tests for external patient surface and bony anatomy reproducibility were performed on both treatment modalities, at the linear accelerator for EBRT and at the MRI for HT, without the use of skin marks, lasers or table shifts to align the volunteer.

### 2. Materials and Methods

#### *2.1 Device production and quality assurance*

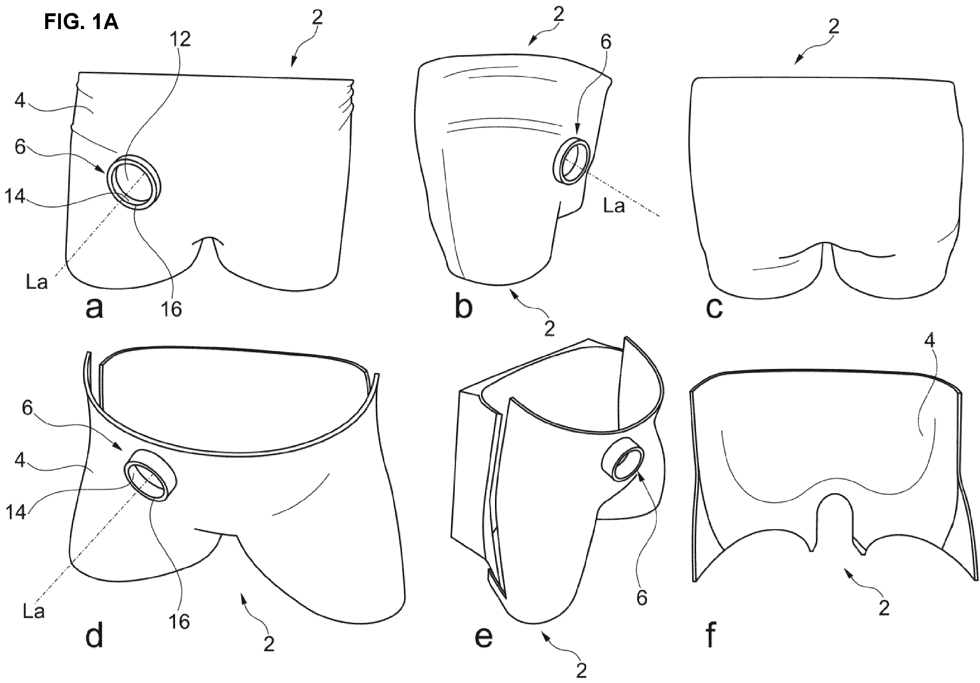

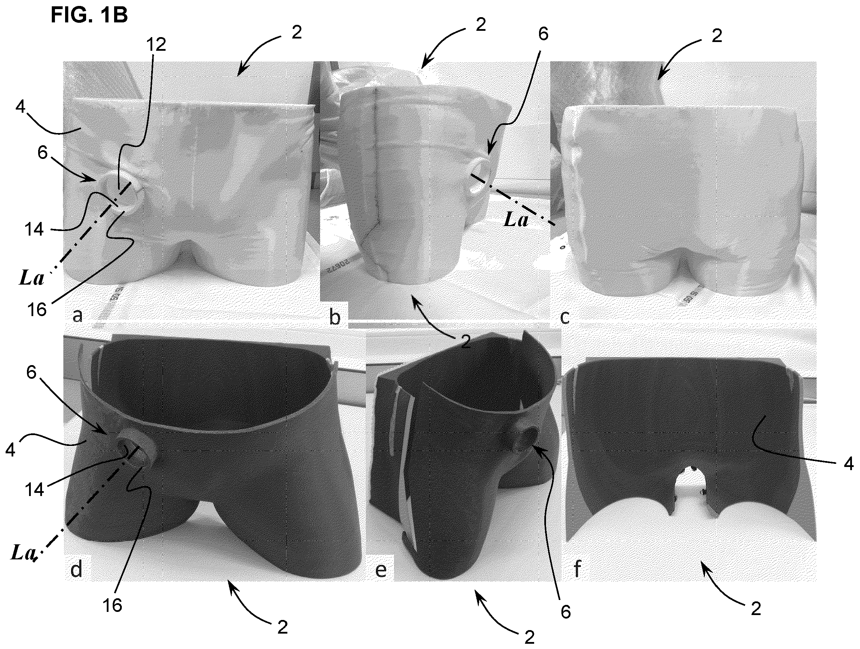

Using a dedicated table, made of non-scattering organic polymer compatible with the surface scanner wavelength, volunteers were placed in a supine position reproducing standard positioning for pelvic EBRT treatments. Knee and ankle support were available per need. US reference images were acquired in this position using a conventional US imaging probe (CHISON Medical Technologies, Jiangsu, China, Sonobook 6, P2-V probe) maintained still by an articulated arm under the supervision of an expert radiologist. Abdominal pulsed color Doppler imaging focusing on the right iliac artery was used as internal organ landmark. The pelvic region and the US imaging probe were modelled with the help of images acquired with a portable metrology grade surface-scanner (HandySCAN™ 300, Creaform, Canada). Its main acquisition parameters are following: optical resolution = 0.025 mm, mesh resolution = 0.1 mm, sampling rate = 205 kHz, light source = 3 lasers diodes, stand-off distance = 300 mm, depth of field = 250 mm. The resulting surface image was cleaned and extruded to a thickness of 5mm using the accompanying software VXelements (Creaform, Canada). First tests of the scanner were previously reported [34]. A 3D model prototype device (Fig. 1), bearing the information of the US probe's angulation and position was created. An MRI compatible and radiation-resistant acrylonitrile-butadiene-styrene (ABS) material was used to print the first device using an A2v4 printer (3NTR, Oleggio, Italy).

To assess the quality of all 3D-printed devices, the printed objects were scanned using the same high-accuracy HandySCAN™ surface scanner used for the model creation. These scans where then semiautomatically aligned and compared with the models used to create the devices using the scanner's accompanying software VxElements to calculate and report a surface deviation distribution. A computed tomography (CT) [35] scan (Philips Big Bore, Utrecht, The Netherlands) of the empty device was acquired with a 1 mm slice thickness and the external device surface was delineated. A dummy radiotherapy (RT) plan was created from this CT scan data and used together with the device contour in order to create a dummy reference surface for the optical surface monitoring (OSM) system (AlignRT™, VisionRT, London, UK). This dummy surface was not used for testing on the volunteer but only to allow opening the AlignRT™ software in the linear accelerator (LINAC) treatment room (Truebeam, Varian Medical systems, Palo Alto, US). However, as also foreseen for final clinical application, the CT scan allows to check the internal quality and homogeneity of the device by visual inspection and by looking at the Hounsfield Units (HU) properties of the device's contour. This is drawn automatically by selecting a HU window level. A minimum of -650 HU was chosen so that the shell thickness corresponded to its physical thickness.

## 2.2 Measurements procedures

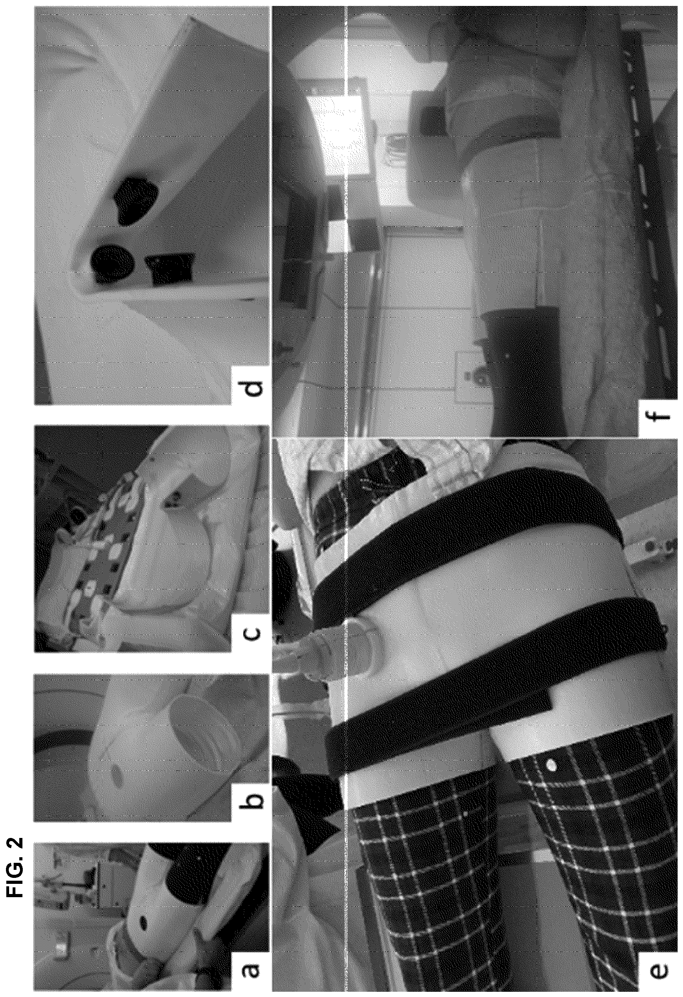

In order to attach the support shell to the LINAC table, a standard Vac-Lok™ cushion fixed onto the couch (Fig. 2a) was molded, using the Vac-Lok™ cushion position as reference frame for the position measurements. The CT image acquisition of the empty device (Fig. 2b) was used for the RT dummy plan. A similar immobilization system was adopted for the MRI table using a more compact cushion (Fig. 2c) because the Vac-Lok™ system was too large and collided with the MRI ring. Three MRI compatible aqueous gel markers were fixed on the bottom segment of the shell to check for movements of the volunteer with respect to the shell, and of the shell with respect to the MRI table (Fig. 2d). US images were acquired outside the MR room (Fig. 2e.).

Moreover, an experimentation was added to evaluate the accuracy of patient positioning without a support shell. As there is no repositioning system for MRI in clinical routine, to show the added value of our system, a technique similar to tattooing in radiotherapy has been used. On the same volunteers as previous, two landmarks were placed on the patient's body (right and left hip) and two landmarks on the MRI table. Then the volunteer was asked to position himself on the table. Two operators further helped him to get the position of best registration between patient landmark and MRI table landmark, direction head-feet and cranio-caudal, respectively. The skin-to-table landmark alignment was visually confirmed within better than 1 mm precision. Furthermore, an MRI marker was attached to the MRI table to check for the table-patient positon while imaging.

To measure the positioning reproducibility achievable with the customized 3D-printed support shell, an OSM system was used for three measurement sessions at the LINAC, followed by a 3 T MRI acquisition (Magnetom Prisma Fit, Siemens, Erlangen, Germany). Each measurement session (OSM or MRI), was composed of 6 or 7 repeated measurement sequences in which the volunteer was asked to position himself within the bottom shell of the device. The top part was then added. After removal of the top part of the device, an image of the volunteer's body shape was acquired with the OSM system (Fig. 2f) and used as reference for measuring shifts between positioning tests.

Between each measurement the volunteer got out of the support shell and then repositioned himself in it. No lasers or skin marks were used to align the volunteer on the LINAC or MRI table, always positioned with the same vertical, longitudinal and lateral coordinates. Measurement sessions 2 and 3 were performed on the same day one week after the first one, while the session at MRI and in the medical room was performed some weeks later.

Using the sub-millimetric positioning resolution of the AlignRT™ system and the LINAC couch [36, 37], three position offsets (vertical, horizontal, and longitudinal) and three rotation angles (yaw, roll, and pitch) were registered for each one of the three LINAC sessions, using the first volunteer positioning of the day as a reference.

For the MRI sessions, with and without support shell, the position differences between the reference 3D image and the other acquisition sequences were calculated on the Eclipse™ treatment planning system (TPS) (Varian medical systems, Palo Alto, US) by collecting the matrix registration parameters (translations and rotations). Using an automatic algorithm of the TPS, the co-registration between images acquisitions was made on the bony anatomy. The 4th image sequence was used as reference image of the session in order to consider anatomical pelvic changes (bladder and rectal filling) potentially occurring during the approximately 40 minutes long acquisition (for the 7 images) that might have made the volunteer uncomfortable and therefore less reproducible in positioning. The main parameters of the T1-weighted turbo spin echo (TSE) MR sequence acquired during each image acquisition were: TR = 784 ms, TE = 12 ms, BW = 170 Hz/pixel, TSE factor = 3, refocusing pulse 180°, physical resolution 1.56 mm x 1.17 mm x 3 mm, GRAPPA = 2, FOV = 300 mm, phase encoding direction HF, phase oversampling = 80%, NSA = 2, TA = 185 s, coil combination Spine and Body matrix (N=30 elements). In addition, the distance between the MRI markers attached to the shell and the lower anterior corner of the sacral body S1 was measured on the sagittal plan by an expert radiologist (JG).

The three dimensional vector of the re-positioning errors was calculated for both OSM and MRI. To compare the 3D vectors length between OSM data and MRI data the Mann-Whitney test was used, with p-values < 0.05 considered statistically significant.

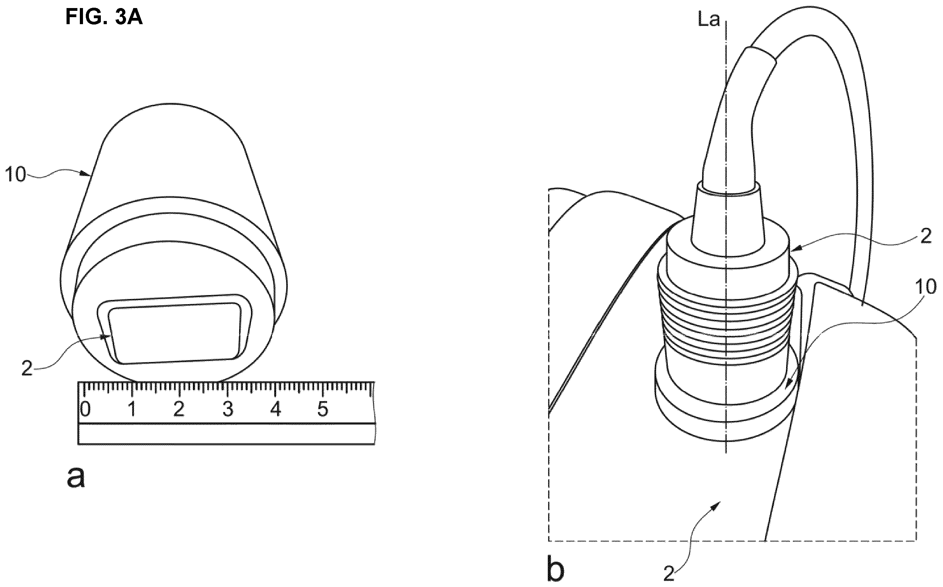

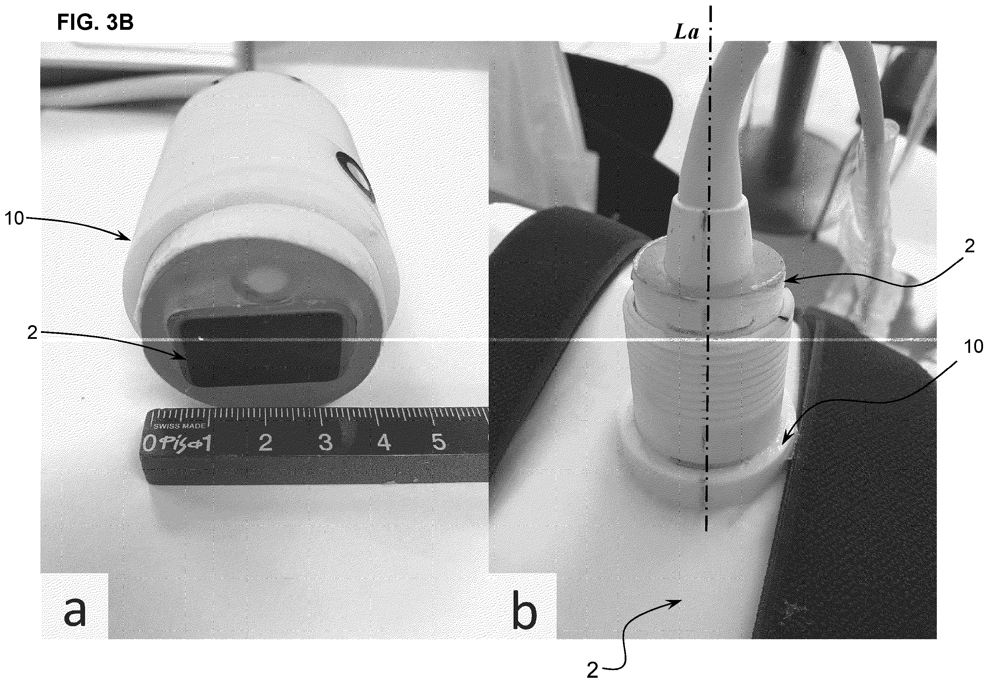

For the US imaging session, the two volunteers were installed in the same position as during the 3D surface scan. An elastic belt was wrapped around the two-part support shell. The US probe was inserted in the locator of the support shell (Fig. 3a and 3b). US images were acquired and compared with the reference images (made prior the creation of the support shell) using a visual depth scaling (cm) ruler present on the US images to check for positioning accuracy between images.

The time required for the volunteer to find the optimal position was registered and analyzed after dichotomization in two categories (≤ 1 or > 1 minute). All volunteers had signed an informed consent by each patient in order to enroll them in the study and collect their data.

### 3. Results

The printing time of the support shell for the first volunteer was 65 hours and the device, split in two-parts, measured approximatively 35 cm x 33 cm x 12 cm (per piece), for a total weight of 1.3 kg. For the second device the printing time was 77 hours, measured approximately 51.5 cm x 35 cm x 16 cm (per piece) for a total weight of 1.7 kg. The volunteers were chosen with two different body mass indexes (BMIs of 22 and 31 for volunteer 1 and 2, respectively) to prove that the 3D immobilization system can be adapted to different types of morphology.

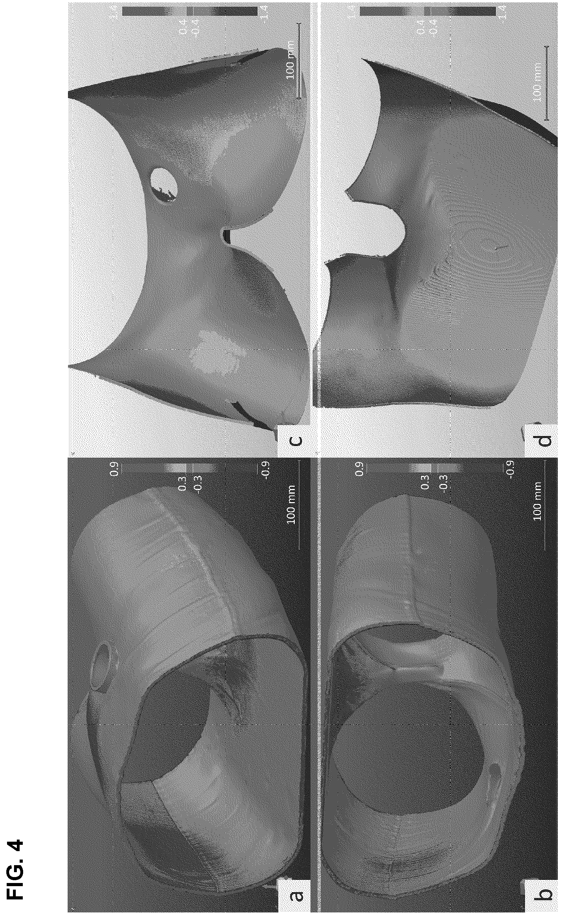

The CT scan density of the support shell was uniform for all volunteers, with a mean Hounsfield unit (HU) number of -350 (± 125, standard deviation). Comparison on the VxElements™ platform between the original models used for 3D-printing and the surface scans of the printed parts of two volunteers are illustrated on Fig. 4. The distribution of surface deviations is almost Gaussian, with a mean of zero for both volunteers (standard deviation of 0.3 mm and maximum deviations of less than 0.7 mm for the first volunteer and standard deviation of 0.5 and maximum deviations of less than 1.0 mm for the second).

As measured by the OSM system at the LINAC, calculated set-up errors in the vertical and lateral directions over all measurement sessions were minimal, with a median value smaller than 1 mm and a range of about 2 mm for both volunteers (Table 1). In the longitudinal direction, which corresponds to the axis along the patient body, deviations were larger with a median of 4.4 mm and a range of 18.8 mm for the first volunteer (BMI=22). Rotation values in the three axes were small with median values inferior to 1/3 of a degree and a range of about 1 degree. For the second volunteer (BMI=31), the longitudinal deviations were smaller than 0.7 mm, with a range of 1.2mm due to a better fixation on the couch. Rotations values in the three axes were larger, with a median of almost 1 degree and a range of 1.5 degree. The increase in rotation values was potentially due to the weight loss of the volunteer between the 3D scan and the measure with the OSM system (BMI: 31.4 -> BMI: 29.8).

Table 1. Pre-positioning deviations from repeated fitting sessions at the LINAC for two volunteers.

| Measurement Sequence at LINAC | Volunteer 1 (BMI=22) | | | | | | | Volunteer 2 (BMI=31) | | | | | | |
|---|---|---|---|---|---|---|---|---|---|---|---|---|---|---|
| | vertical mm | lateral mm | longitudinal mm | yaw ° | roll ° | pitch ° | 3D vector mm | vertical mm | lateral mm | longitudinal mm | yaw ° | roll ° | pitch ° | 3D vector mm |
| | All measurements | | | | | | | All measurements | | | | | | |
| Median | 0.2 | 0.7 | 4.4 | -0.1 | 0.1 | - 0.3 | 4.5 | -0.5 | 0.2 | 0.7 | 0.8 | 0. 5 0. | -1.2 | 0.8 |
| Min | -0.4 | -0.7 | -4.2 | -0.6 | -0.2 | - 1.2 | 1.2 | -0.7 | -0.1 | -0.1 | 0.3 | 3 | -1.7 | 0.5 |
| Max | 1.2 | 2.2 | 14.6 | 0.3 | 0.4 | 0.5 | 14.6 | 0.1 | 0.6 | 1.1 | 1.3 | 0. 7 | -0.2 | 1.1 |

Example of repositioning results, and using the device, as assessed on MRI for a volunteer are shown in Table 2. By using the 3D-printed device, the reproducibility based on the bony anatomy was excellent for both volunteers, with only small lateral and vertical deviations with a median value of less than 0.5 mm and a range of about 2.5 mm for the first volunteer and a median value of 0.5mm with a range of 3.8 mm for the second. In analogy to the LINAC measurements, longitudinal deviations were the largest, with a median of 1.4 mm and a range of 8.5 mm for the first volunteer and a median of 1.4mm and a range of 7.5 mm for the second. Rotations were small with a median value near to zero and a range of about 1° for the first volunteer and a median near 0.5° with a range of about 2° for the second.

Table 2. Pre-positioning deviations of the internal bony anatomy from repeated fitting session of two volunteers as measured using Magnetic Resonance Imaging using the support shell.

| | Volunteer 1 (BMI=22) | | | | | | | Volunteer 2 (BMI=31) | -0.5 | -1.4 | 1.1 | 0.1 | -0.2 | 2.3 |
|---|---|---|---|---|---|---|---|---|---|---|---|---|---|---|
| Measurement Sequence at MRI | vertical mm | lateral mm | longitudinal mm | yaw ° | roll ° | pitch ° | 3D vector mm | vertical mm | lateral mm | longitudinal mm | yaw ° | roll ° | pitch ° | 3D vector mm |
| All measurements (with device) | | | | | | | | All measurements (with device) | | | | | | |
| Min | -0.4 | -1.3 | -7.4 | -0.7 | -0.1 | - 0.6 | 0.5 | -2.2 | -2.8 | -4.1 | -1.6 | 0.7 | -1.4 | 1.4 |
| Max | 2.0 | 0.3 | 1.1 | 0.1 | 0.4 | 0.6 | 7.7 | 1.6 | 2.6 | 3.4 | 0.3 | 1.3 | 0.4 | 5.4 |

When analyzing the three dimensional vector values obtained with the two measurement modalities (OSM and MRI), the difference between the two median values was larger for the first volunteer, 4.5 mm versus 1.5 mm for the OSM and MRI technique, and smaller, 0.8 mm versus 2.3 mm for the second volunteer.

Complimentary, on the 7 MRI acquisitions the calculated mean (SD) distance between the MRI markers on the shell and the lower anterior corner of the vertebral body S4 was 210.7 (1.8) mm for the first volunteer and the calculated mean (SD) distance between the MRI markers on the shell and the lower anterior corner of the vertebral body S2 was 196.4 (1.4) mm for the second volunteer. These results confirmed that patient's bone anatomy position was reproducible with respect to the rigid shell itself with millimeter accuracy.

Repositioning results determined on MRI for the two volunteers without support shell are shown in Table 3. As expected, the treatment would not be accurate, the median modulus of the computed 3D displacement vector is 4 to 6 times larger without support shell than with device. This condition was confirmed by the large SD of the measured distance between the MRI marker on the MR table and the lower anterior corner of the vertebral body S4 without device, 9.5 mm for the first volunteer and 6.6 mm for the second.

Table 3. Pre-positioning deviations of the internal bony anatomy from repeated fitting session of two volunteers as measured using Magnetic Resonance Imaging without support shell.

| | Volunteer 1 (BMI=22) | | | | | | | Volunteer 2 (BMI=31) | | | | | | |
|---|---|---|---|---|---|---|---|---|---|---|---|---|---|---|
| Measurement Sequence at MRI | vertical mm | lateral mm | longitudinal mm | yaw ° | roll ° | pitch ° | 3D vector mm | vertical mm | lateral mm | longitudinal mm | yaw ° | roll ° | pitch ° | 3D vector mm |
| All measurements (without device) | | | | | | | | All measurements (without device) | | | | | | |
| Median | -1.5 | -5.6 | -4.6 | -0.8 | 0.2 | 0.1 | 9.8 | -1.1 | 2.0 | -8.2 | 0.5 | 0.7 | -0.2 | 8.5 |
| Min | -1.9 | -9.0 | -6.5 | -1.5 | -0.1 | - 0.8 | 4.6 | -1.4 | -3.0 | -12.1 | 0.0 | 0.5 | -1.1 | 6.8 |
| Max | 2.9 | -2.0 | 8 | 0.5 | 0.6 | 0.6 | 10.5 | 0.2 | 8.0 | -5.4 | 1.1 | 1.0 | 0.7 | 14. 5 |

The time needed for the two volunteers to self-align in the support shell and accommodate was always less than 1 minute for all of the measurement sequences (n= 28).

Finally, the agreement in target repositioning was high between the US reference image from the surface scanning setup and the ulterior image acquired with the US probe embedded in the customized 3D device: the right iliac vessels were in the same anatomic plane, visualized at similar depth within 2mm precision. This is a measure of how a US HT applicator could appropriately target the region to heat up.

4. Discussion

MRgFUS-based deep HT represents a promising technology for improving the therapeutic ratio of curative EBRT treatments [18, 29]. In this study, with our 3D-printed pelvic support shell, the position of volunteers on both the LINAC and the MRI was reproducible.

Tests indicated positioning errors monitored at the LINAC by the OSM system were millimetric, with body rotations mainly within 0.5°. Of note, these results were obtained by positioning the couch always at the same spatial coordinates, with no use of lasers or marks on the support shell. On the other hand, as already demonstrated in the literature [38], the largest positioning errors was in the longitudinal axis. By limiting rotational movements, improvements in daily reproducibility may be expected especially for LINACs equipped with a fixed flat couch that do not allow rotational corrections, like on MR-LINACS. Noteworthy, uncorrected rotational errors may have a significant effect on organ sparing, as observed for example for the rectal $V50_{Gy}$ dose parameter in prostate cancer patients [39].

The MRI tests showed the potential ability of the support shell to ensure a bony-based inter-fractional repositioning with millimetric accuracy (Table 2) and limited longitudinal movements. In addition, by fixing the patient on the couch, improvements in reducing the intra-fraction motion of the patient during the EBRT and HT sessions can be expected using such a device. On the other hand, as expected, the internal organ displacement was greater over time as observed during the 40-minutes MRI acquisition time, mainly due to the bladder and rectum filling [30]. Implementation in the daily clinical practice of dedicated rectal and bladder preparation protocols is expected to improve the intra-fraction reproducibility of HT treatments.

The OSM measuring procedure showed larger pre-positioning errors compared to the MRI results. This difference between the two measuring procedures was likely due to the error induced by the physiological breathing when using the OSM system, as previously shown by Li *et al.* [40].

With this study the possibility of embedding an US applicator that should allow a fast and reliable positioning from one HT session to another was demonstrated, reducing patient movements and optimizing treatment time and resources. As a proof of concept and for workflow convenience, abdominal ultrasonography was used here. With respect to the US beam application for induction of deep HT in the prostate tumor bed, a transperineal acoustic window similar to commercially available transperineal 4D ultrasound systems [9] will be used, however at higher level of applied acoustic energy. For the envisaged configuration, the direction of propagation of the acoustic beam will mainly be parallel to the longitudinal axis corresponding to a smooth attenuation profile, and therefore the positioning error of the applicator relative to the patient pelvis in the longitudinal axis would translate into minimal effects on the thermal build up control. The requirement for positioning accuracy is significantly higher in the vertical and lateral directions corresponding to steeper thermal gradients, whereas the beam direction needs to be controlled by electronic steering or mechanical rotation of the applicator. The reported accuracies in this study (lateral/vertical median 0.5mm and longitudinal median 1.5mm) are consistently compliant with the estimated needs for HIFU-induced deep HT.

To the best of our knowledge, no 3D-printed pelvic support shell integrating a specific US applicator holder have been described in the literature for combined EBRT and MRgFUS deep HT treatments. The complexity of creating a model that should fit a deformable region like the pelvis can represent a major challenge. The quality assurance tests performed on the device confirmed its precision despite the large size of the support shells. The largest deviations were observed at the outer edges and the middle of the shell (Fig. 4). The thickness of the printed shell is suggested in a range from 3-7mm, for instance around 5 mm, to provide sufficient rigidity.

The support shell allowed for a fast patient self-positioning, within 1 minute without the use of skin marks or lasers. Considering the patient positioning challenges in the laser-free environment of MR-LINACs and the long treatment times for adaptive treatments [41], the device can be particularly interesting to improve set-up times and limit the intra-fraction motion of MR-guided EBRT treatments [42]. In the context of a clinical study, the patient could perform the surface scan for the 3D modelling on the same day that he would have his first consultation with the radiation oncologist, where the treatment plan should be presented to him. The support shell could be manufactured in the following days allowing for the HT probe to be later connected and adjusted in the wanted position. The patient would further return to the hospital to perform a planning CT scan already using the 3D printed device. The HT sessions are planned one hour before the EBRT session. Overall, the patient should not be requested to extra visits to hospital on the top of the standard-of-care workflow. Moreover, the required printing time would probably be alleviated in the future thanks to current research on reducing printing times and the use of several printers working together on different pieces. The high cost in the production of the device could be also alleviated when devices will be produced in larger series or when the department will acquire a 3D printer. Following the results of our study, immobilization of the patient using the support shell with a locator for the HT transducer are advantageous for the reproducibility of the treatment between each session. Moreover, to position the patient identical in both HT and EBRT session, will make fusion of HT and EBRT planning easier. This is why the proposed invention is very interesting for this combined treatment.

## 5. Conclusions

In conclusion, in this study the feasibility of producing a personalized 3D-printed pelvic support shell for use as patient-specific US and HT transducer holder for combined EBRT and MRgFUS deep HT treatments in the pelvic region was demonstrated. The device allowed millimetric positioning accuracy and minimal angular rotations. This device may be considered a promising immobilization support able to ensure a fast patient setup and an optimal inter- and intra-fractional control motion for this combined treatment as well as for modem adaptive EBRT techniques with MR-LINACs.

## *Embodiment of treatment control system and method using the Green function*

In a method for hyperthermia cancer treatment according to an embodiment of the invention, a treatment control system configured to control a treatment process without systematic MRI (magnetic resonance imaging) control for safe and accurate ultrasound-based HT is provided. The reduction of MRI control reduces logistical burden and cost, and therefore, streamlines the HT procedure and allows to provide many radiotherapy settings.

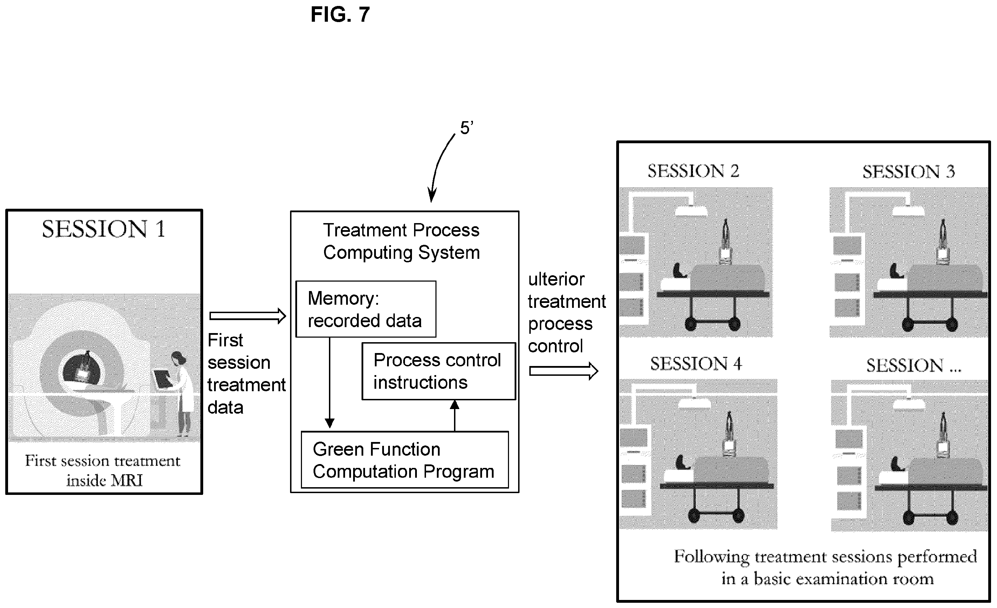

For motion-free situations (bony metastasis, pelvis and breast cancer recurrences etc.), the process enables reproducing the position and the heating pattern without the expensive, time-consuming MRI that also requires dedicated staff. MRI is only needed for the first treatment session. Ulterior HT sessions could be administrated in a basic examination room, as illustrated in figure 7.

This solution further requires tissue temperature prediction. The temperature prediction is a critical success factor to dramatically reduce set-up time for hyperthermia in radiotherapy units and thus makes the system attractive to hospitals. This highly targeted local hyperthermia not only increases precision in temperature delivery but also reduces the potential toxicity related to a non-uniform heating of the target and surrounding tissues.

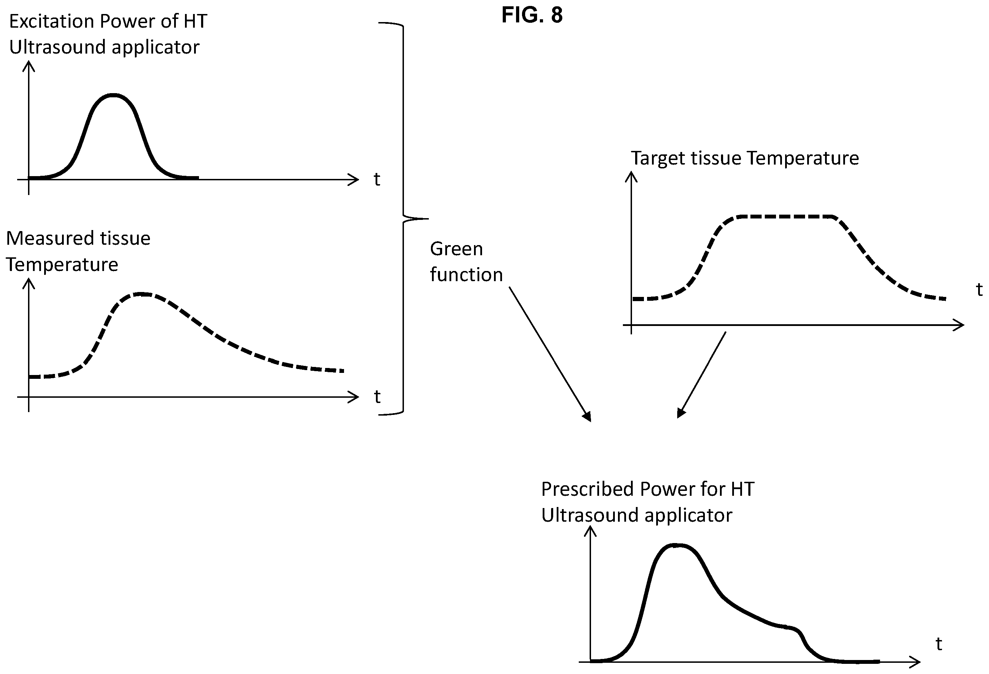

Hyperthermia is typically delivered fractionated: for instance ten, twenty, or even more sessions matching the RT (radiotherapy) protocol. In conventional systems, although performing MR-controlled ultrasound-based hyperthermia is accurate and safe, the costs and logistics in a fractionated approach are dissuasive. In the present invention, according to an embodiment as illustrated in figure 7, the system for radiation with hyperthermia cancer treatment comprises a computing system 5' including a closed loop automatic controller and a program module configured for controlling a treatment process. The treatment process comprises performing a first session of HT under MR control, using the closed loop automatic controller, to record in the computing system the applied acoustic parameters element-wise, and to compute in the computing system the Green function of the treated tissue. Using the Green function calculation, the program module may control execution of a "play back" sonication outside the MR room using dedicated immobilisation devices for fast and accurate treatment "replay" for the remaining 90 to 95% of the treatment sessions. As illustrated in figure 8, the Green function calculation serves to calculate a prescribed power over time, based on a target tissue temperature, for controlling the ultrasound hyperthermia applicator device 8. This approach reduces infrastructure and treatment costs for clinical applications of ultrasound-based hyperthermia.

In an embodiment of the present invention, the process comprises 1) recording of MRI data and log files of the hyperthemia ultrasound device control parameters in the computing system, 2) deconvolution of the data by a program module in the computing system, using the Green function, to compute a prescribed power over time for the HT Ultrasound applicator, 3) providing a treatment device and support shell with treatment device locator to reproduce the position and orientation of the HT ultrasound applicator between the treatment sessions of the same patient, as described previously, and 4) controlling the HT ultrasound applicator using the computed power curve. Advantageously, the provision of the support shell with treatment device locator to accurately reproduce the position and orientation of the HT ultrasound applicator between the treatment sessions of the same patient, combines well with the treatment process computing system including the program module configured for controlling the treatment process, because it ensures that the computed output obtained from the first session data may be used to control subsequent sessions in an accurate reproducible manner. In effect, the assumption underlying the use of the Green function to prescribe the power of the ultrasound HT applicator device is that the position and direction of the HT applicator device remains the same from session to session.

A Green's function is the impulse response of an inhomogenous linear differential operator defined on a domain with specified initial conditions or boundary conditions. The underlying hypothesis in the present process embodiment is that when the temperature elevation is low, the deviation of the system from linearity is also low. Thus, on the assumption that this non-linear system can be approximated by a linear system, one can apply the Green function. A temperature rise error that is systematically inferior to 15% will validate the hyperthermia transducer without MRI control. In the present case, according to process embodiments of the invention, the Green's function is a 4-dimension object (space and time). In optics, the Green function is bi-dimensional and called "point spread function" in the direct image and respectively "optical transfer function" in the Fourier (reciprocal) space.

Theoretically, the Green function should be determined by applying a Dirac delta function-like excitation to the system and measuring the output. However, for practical reasons, this approach is not feasible in a real situation.

In embodiments of the present invention, the program module for HT treatment is configured to excite the system with a time-dependent Gaussian input function and to perform Fourier space deconvolution. The Fourier transformation of the Gaussian function is again a Gaussian function, therefore there are no numerical instabilities in the process.

We write a generic Gaussian function in space and time as

$$Gauss(\sigma_x, \sigma_y, \sigma_z, \tau) = exp\left[-\left(\frac{x^2}{2\sigma_x^2} + \frac{y^2}{2\sigma_y^2} + \frac{z^2}{2\sigma_z^2}\right)\right] \cdot exp\left[-\frac{t^2}{2\tau^2}\right]$$

In the direct space-time (physical space-time), under conditions of linear system, the temperature elevation is :

$$\Delta T^{meas}(\vec{r}, t) = P_0 \cdot Gauss(\sigma_x, \sigma_y, \sigma_z, \tau) \otimes Green(\vec{r}, t)$$

Where $\otimes$ symbol stands for the convolution product.

In the reciprocal space-time (Fourier transformation)

$$FT[\Delta T^{meas}(\vec{r}, t)] = P_0 \cdot 4 \cdot \pi^2 \cdot \sigma_x \cdot \sigma_y \cdot \sigma_z \cdot \tau \cdot Gauss\left(\frac{1}{2\pi \cdot \sigma_x}, \frac{1}{2\pi \cdot \sigma_y}, \frac{1}{2\pi \cdot \sigma_z}, \frac{1}{2\pi \cdot \tau}\right) \cdot FT[Green(\vec{r}, t)]$$

$$Green(\vec{r}, t) = FT^{-1}\left[\frac{FT[\Delta T^{meas}(\vec{r}, t)]}{P_0 \cdot 4 \cdot \pi^2 \cdot \sigma_x \cdot \sigma_y \cdot \sigma_z \cdot \tau \cdot Gauss\left(\frac{1}{2\pi \cdot \sigma_x}, \frac{1}{2\pi \cdot \sigma_y}, \frac{1}{2\pi \cdot \sigma_z}, \frac{1}{2\pi \cdot \tau}\right)}\right]$$

In order to avoid artificial amplification of the experimental noise, the point wise value of the above fraction should be set to zero when the modulus of the numerator is smaller than 5% of its absolute maximum.

Once the Green function is known, given a target temperature evolution $\Delta T^{target}(\vec{r}, t)$, the prescribed acoustic power is determined as :

$$P(\vec{r}, t) = FT^{-1}\left[\frac{FT[\Delta T^{target}(\vec{r}, t)]}{FT[Green(\vec{r}, t)]}\right]$$

$$= P_0 \cdot 4 \cdot \pi^2 \cdot \sigma_x \cdot \sigma_y \cdot \sigma_z \cdot \tau \cdot FT^{-1}\left[Gauss\left(\frac{1}{2\pi \cdot \sigma_x}, \frac{1}{2\pi \cdot \sigma_y}, \frac{1}{2\pi \cdot \sigma_z}, \frac{1}{2\pi \cdot \tau}\right) \cdot \frac{FT[\Delta T^{target}(\vec{r}, t)]}{FT[\Delta T^{meas}(\vec{r}, t)]}\right]$$

In order to avoid artificial amplification of the experimental noise, the point wise value of the fraction should be set to zero when the modulus of the numerator is smaller than 5% of its absolute maximum, or when the modulus of the denominator is smaller than 5% of its absolute maximum.

In some cases, the problem should be reduced to 1D (the time is the only variable). For instance, controlling the temperature at the focal point while not actively intervening on the temperature evolution elsewhere.

The 1D formalism becomes :

$$Gauss(\tau) = exp\left[-\frac{t^2}{2\tau^2}\right]$$

$$P(t) = P_0 \cdot \sqrt{2\pi} \cdot \tau \cdot FT^{-1}\left[Gauss\left(\frac{1}{2\pi \cdot \tau}\right) \cdot \frac{FT[\Delta T^{target}(t)]}{FT[\Delta T^{meas}(t)]}\right]$$

The target temperature elevation $\Delta T^{target}(t)$ needs to fulfill some conditions of regularity in order to enable the existence of the $P(t)$ as a real positive function. Typically it should be of class C2 and should not vary more rapidly than the characteristic response time of the system that is empirically known from $\Delta T^{meas}(t)$.

**Prior Literature References**

1. Ssrpm. Report number 11, Revision 2014. Quality control of medical electron accelerators. ISBN 3 908 125 57 X ( 2014).

2. Carrie C, Magne N, Burban-Provost P et al. Short-term androgen deprivation therapy combined with radiotherapy as salvage treatment after radical prostatectomy for prostate cancer (GETUG-AFU 16): a 112-month follow-up of a phase 3, randomised trial. Lancet Oncol 20(12), 1740-1749 (2019).

3. Achard V, Panje CM, Engeler D, Zilli T, Putora PM. Localized and Locally Advanced Prostate Cancer: Treatment Options. Oncology doi:10.1159/000513258 1-9 (2021).

4. Tendulkar RD, Agrawal S, Gao T et al. Contemporary Update of a Multi-Institutional Predictive Nomogram for Salvage Radiotherapy After Radical Prostatectomy. J Clin Oncol 34(30), 3648-3654 (2016).

5. Dal Pra A, Panje C, Zilli T et al. Salvage radiotherapy for macroscopic local recurrences after radical prostatectomy : A national survey on patterns of practice. Strahlenther Onkol 194(1), 9-16 (2018).

6. Zilli T, Jorcano S, Peguret N et al. Results of Dose-adapted Salvage Radiotherapy After Radical Prostatectomy Based on an Endorectal MRI Target Definition Model. Am J Clin Oncol 40(2), 194-199 (2017).

7. Zilli T, Jorcano S, Peguret N et al. Dose-adapted salvage radiotherapy after radical prostatectomy based on an erMRI target definition model: toxicity analysis. Acta Oncol 53(1), 96-102 (2014).

8. Picardi C, Perret I, Miralbell R, Zilli T. Hypofractionated radiotherapy for prostate cancer in the postoperative setting: What is the evidence so far? Cancer Treat Rev 62 91-96 (2018).

9. Richter A, Exner F, Weick S et al. Evaluation of intrafraction prostate motion tracking using the Clarity Autoscan system for safety margin validation. Z Med Phys 30(2), 135-141 (2020).

10. Dewhirst MW, Vujaskovic Z, Jones E, Thrall D. Re-setting the biologic rationale for thermal therapy. Int J Hyperthermia 21(8), 779-790 (2005).

11. Datta NR, Puric E, Klingbiel D, Gomez S, Bodis S. Hyperthermia and Radiation Therapy in Locoregional Recurrent Breast Cancers: A Systematic Review and Meta-analysis. Int J Radiat Oncol Biol Phys 94(5), 1073-1087 (2016).

12. Datta NR, Rogers S, Ordonez SG, Puric E, Bodis S. Hyperthermia and radiotherapy in the management of head and neck cancers: A systematic review and meta-analysis. Int J Hyperthermia 32(1), 31-40 (2016).

13. Datta NR, Stutz E, Gomez S, Bodis S. Efficacy and Safety Evaluation of the Various Therapeutic Options in Locally Advanced Cervix Cancer: A Systematic Review and Network Meta-Analysis of Randomized Clinical Trials. Int J Radiat Oncol Biol Phys 103(2), 411-437 (2019).

14. Crezee J, Van Leeuwen CM, Oei AL et al. Biological modelling of the radiation dose escalation effect of regional hyperthermia in cervical cancer. Radiat Oncol 11 14 (2016).

15. Beck M, Ghadjar P, Mehrhof F et al. Salvage-Radiation Therapy and Regional Hyperthermia for Biochemically Recurrent Prostate Cancer after Radical Prostatectomy (Results of the Planned Interim Analysis). Cancers (Basel) 13(5), (2021).

16. Kok HP, Crezee J, Franken NA, Stalpers LJ, Barendsen GW, Bel A. Quantifying the combined effect of radiation therapy and hyperthermia in terms of equivalent dose distributions. Int J Radiat Oncol Biol Phys 88(3), 739-745 (2014).

17. Guillemin PC, Gui L, Lorton O et al. Mild hyperthermia by MR-guided focused ultrasound in an ex vivo model of osteolytic bone tumour: optimization of the spatio-temporal control of the delivered temperature. J Transl Med 17(1), 350 (2019).

18. Zhu L, Lam D, Pacia CP et al. Characterization of magnetic resonance-guided high-intensity focused ultrasound (MRgHIFU)-induced large-volume hyperthermia in deep and superficial targets in a porcine model. Int J Hyperthermia 37(1), 1159-1173 (2020).

19. Baust JM, Rabin Y, Polascik TJ et al. Defeating Cancers' Adaptive Defensive Strategies Using Thermal Therapies: Examining Cancer's Therapeutic Resistance, Ablative, and Computational Modeling Strategies as a means for Improving Therapeutic Outcome. Technol Cancer Res Treat 17 1533033818762207 (2018).

20. Jolesz FA. MRI-guided focused ultrasound surgery. Annu Rev Med 60 417-430 (2009).

21. Melodelima D, Salomir R, Mougenot C et al. Intraluminal ultrasound applicator compatible with magnetic resonance imaging "real-time" temperature mapping for the treatment of oesophageal tumours: an ex vivo study. Med Phys 31(2), 236-244 (2004).

22. Moonen CT, Quesson B, Salomir R et al. Thermal therapies in interventional MR imaging. Focused ultrasound. Neuroimaging Clin N Am 11(4), 737-747, xi (2001).

23. Zhou YF. High intensity focused ultrasound in clinical tumor ablation. World J Clin Oncol 2(1), 8-27 (2011).

24. Marien A, Gill I, Ukimura O, Betrouni N, Villers A. Target ablation--image-guided therapy in prostate cancer. Urol Oncol 32(6), 912-923 (2014).

25. Rueff LE, Raman SS. Clinical and Technical Aspects of MR-Guided High Intensity Focused Ultrasound for Treatment of Symptomatic Uterine Fibroids. Semin Intervent Radiol 30(4), 347-353 (2013).

26. Ellis S, Rieke V, Kohi M, Westphalen AC. Clinical applications for magnetic resonance guided high intensity focused ultrasound (MRgHIFU): present and future. J Med Imaging Radiat Oncol 57(4), 391-399 (2013).

27. Guilhon E, Quesson B, Moraud-Gaudry F et al. Image-guided control of transgene expression based on local hyperthermia. Mol Imaging 2(1), 11-17 (2003).

28. Zhu L, Huang Y, Lam D et al. Targetability of cervical cancer by magnetic resonance-guided high-intensity focused ultrasound (MRgHIFU)-mediated hyperthermia (HT) for patients receiving radiation therapy. Int J Hyperthermia 38(1), 498-510 (2021).

29. Zhu L, Partanen A, Talcott MR et al. Feasibility and safety assessment of magnetic resonance-guided high-intensity focused ultrasound (MRgHIFU)-mediated mild hyperthermia in pelvic targets evaluated using an in vivo porcine model. Int J Hyperthermia 36(1), 1147-1159 (2019).

30. Ghadjar P, Fiorino C, Munck Af Rosenschold P, Pinkawa M, Zilli T, Van Der Heide UA. ESTRO ACROP consensus guideline on the use of image guided radiation therapy for localized prostate cancer. Radiother Oncol 141 5-13 (2019).

31. Moser T, Creed M, Walker R, Meier G. Radiotherapy tattoos: Women's skin as a carrier of personal memory-What do we cause by tattooing our patients? Breast J 26(2), 316-318 (2020).

32. Sun J, Pichler P, Dowling J et al. MR simulation for prostate radiation therapy: effect of coil mounting position on image quality. Br J Radiol 87(1042), 20140325 (2014).

33. Feddersen TV, Hemandez-Tamames JA, Franckena M, Van Rhoon GC, Paulides MM. Clinical Performance and Future Potential of Magnetic Resonance Thermometry in Hyperthermia. Cancers (Basel) 13(1), (2020).

34. Dipasquale G, Poirier A, Sprunger Y, Uiterwijk JWE, Miralbell R. Improving 3D-printing of megavoltage X-rays radiotherapy bolus with surface-scanner. Radiat Oncol 13(1), 203 (2018).

35. Fahimian B, Yu V, Horst K, Xing L, Hristov D. Trajectory modulated prone breast irradiation: A LINAC-based technique combining intensity modulated delivery and motion of the couch. Radiotherapy and Oncology 109(3), 475-481 (2013).

36. Schmidhalter D, Fix MK, Wyss M et al. Evaluation of a new six degrees of freedom couch for radiation therapy. Med Phys 40(11), 111710 (2013).

37. Schoffel PJ, Harms W, Sroka-Perez G, Schlegel W, Karger CP. Accuracy of a commercial optical 3D surface imaging system for realignment of patients for radiotherapy of the thorax. Phys Med Biol 52(13), 3949-3963 (2007).

38. Bartoncini S, Fiandra C, Ruo Redda MG, Allis S, Munoz F, Ricardi U. Target registration errors with surface imaging system in conformal radiotherapy for prostate cancer: study on 19 patients. Radiol Med 117(8), 1419-1428 (2012).

39. Chiesa S, Placidi L, Azario L et al. Adaptive optimization by 6 DOF robotic couch in prostate volumetric IMRT treatment: rototranslational shift and dosimetric consequences. J Appl Clin Med Phys 16(5), 35-45 (2015).

40. Li W, Jiang Z, Chu K, Jin J, Ge Y, Cai J. A Noninvasive Method to Reduce Radiotherapy Positioning Error Caused by Respiration for Patients With Abdominal or Pelvic Cancers. Technol Cancer Res Treat 18 1533033819825865 (2019).

41. Mcpartlin AJ, Li XA, Kershaw LE et al. MRI-guided prostate adaptive radiotherapy - A systematic review. Radiother Oncol 119(3), 371-380 (2016).

42. Grimwood A, Mcnair HA, O'shea TP et al. In Vivo Validation of Elekta's Clarity Autoscan for Ultrasound-based Intrafraction Motion Estimation of the Prostate During Radiation Therapy. Int J Radiat Oncol Biol Phys 102(4), 912-921 (2018).

43. Wang L, Cmelak AJ, Ding GX. A simple technique to improve calculated skin dose accuracy in a commercial treatment planning system. J Appl Clin Med Phys 19(2), 191-197 (2018).

### *List of references in the drawings:*

System for radiation with hyperthermia cancer treatment 1
Patient support device
Scanning system 3
Scanner
Computing system 5
Image processing software
Treatment process computing system 5'
HT treatment program module
Green function computation program
Support shell 2

    Body conforming wall 4
    Treatment device locator 6

        Orifice 12
        Guide tube 14

           Rim
           outer edge 16

Support shell manufacturing machine 7
3D printing machine

Treatment device 8, 8a, 8b

    Hyperthermia applicator (single channel or multi-channel)
    Radiation emitter
    Monitoring probe
    Device adaptor 10
    Coupling means (not shown)

**Claims**

1. **System for radiation with hyperthermia cancer treatment** (1) comprising a rigid support shell (2), the support shell comprising a body conforming wall (4) and at least one treatment device locator (6),
the treatment device locator comprising an orifice (12) and a guide tube (14) upstanding outwardly from the body conforming wall and configured to receive and locate a treatment device (8) including an ultrasound hyperthermia applicator (8a), wherein the system further comprises a treatment process computing system (5') including a program module configured for controlling a treatment process in which data of a first session of HT under MR control is recorded in the computing system, the program module comprising a Green function computation module configured to compute a prescribed power over time, based on said recorded data of a first session and a target tissue temperature, for outputting process control data for controlling the ultrasound hyperthermia applicator in subsequent sessions.

2. System according to the preceding claim wherein the support shell comprises at least two separable parts removably fixable together for positioning around a body part of a patient.

3. System according to the preceding claim further comprising a device adaptor (10) having an adjustable or tunable interface coupling to the guide tube of the treatment device locator and an inner interface locating the treatment device.

4. System according to any preceding claim further comprising said treatment device which includes a radiation emitter (8b).

5. System according to any preceding claim further comprising said treatment device which includes a monitoring device.

6. System according to any preceding claim wherein the support wall consists of or is principally made of a polymer material, preferably selected from a group consisting of Acrylonitrile Butadiene Styrene, Polylactic Acid and Nylon.

7. System according to any preceding claim wherein the support wall has an average thickness in a range of 1-7mm.

8. System according to the preceding claim wherein the support wall has an average thickness in a range of 3-7mm.

9. System according to any preceding claim further comprising a computing system (5) and image processing software installed therein configured to generate a 3-dimensional form of the support shell and to further generate a manufacturing file from the 3-D form of the support shell for transmission to a support shell manufacturing machine.

10. System according to any preceding claim wherein the system further comprises a support shell manufacturing machine (7) including an additive manufacturing device, for instance a 3D printing device.

11. System according to any preceding claim wherein the guide tube is cylindrical.

12. System according to any preceding claim wherein the treatment device locator and the treatment device optionally with device adaptor (10) couple together defining a specific position on the support shell and a specific target therapy direction ($La$) relative to the support shell.

13. System according to the preceding claim wherein the treatment device locator and treatment device, optionally with device adaptor (10), interface with a pre-defined amount of play allowing a specified degree of freedom in the direction of the treatment device relative to the axis of treatment ($La$), the adjustment being within a cone having an angle of less than 10°.

14. System according to the preceding claim wherein said adjustment is within a cone having an angle of less than 5°.

**Patentansprüche**

1. System zur Bestrahlung mit Hyperthermie-Krebsbehandlung (1), die einen starren Stützmantel (2) umfasst, wobei der Stützmantel eine sich einem Körper anpassende Wand (4) und mindestens eine Behandlungsvorrichtungspositioniereinrichtung (6) umfasst, wobei die Behandlungsvorrichtungspositioniereinrichtung eine Öffnung (12) und ein Führungsrohr (14) umfasst, das von der sich dem Körper anpassenden Wand nach außen hochsteht und dazu ausgestaltet ist, eine Behandlungsvorrichtung (8) aufzunehmen und zu positionieren, die einen Ultraschallhyperthermie-Applikator (8a) umfasst, wobei das System ferner ein Behandlungsprozess-Rechensystem (5') umfasst, das ein Programmmodul umfasst, das zum Steuern eines Behandlungsprozesses ausgestaltet ist, in dem Daten einer ersten HT-Sitzung unter MR-Steuerung in dem Rechensystem aufgezeichnet werden, wobei das Programmmodul ein Greensche-Funktion-Berechnungsmodul umfasst, das dazu ausgestaltet ist, eine vorgeschriebene Leistung über die Zeit auf der Grundlage der aufgezeichneten Daten einer ersten Sitzung und einer Zielgewebetemperatur zu berechnen, um Prozesssteuerdaten zum Steuern des Ultraschallhyperthermie-Applikators in nachfolgenden Sitzungen auszugeben.

2. System nach dem vorhergehenden Anspruch, wobei der Stützmantel mindestens zwei trennbare Teile umfasst, die zum Positionieren um einen Körperteil eines Patienten abnehmbar zusammen befestigbar sind.

3. System nach dem vorhergehenden Anspruch, ferner umfassend einen Vorrichtungsadapter (10), der eine anpassbare oder einstellbare Grenzfläche, die sich an das Führungsrohr der Behandlungsvorrichtungspositioniereinrichtung koppelt, und eine innere Grenzfläche aufweist, welche die Behandlungsvorrichtung positioniert.

4. System nach einem der vorhergehenden Ansprüche, ferner umfassend die Behandlungsvorrichtung, die einen Strahlungsemitter (8b) umfasst.

5. System nach einem der vorhergehenden Ansprüche, welche ferner die Behandlungsvorrichtung umfasst, die eine Überwachungsvorrichtung umfasst.

6. System nach einem der vorhergehenden Ansprüche, wobei die Stützwand aus einem Polymermaterial besteht oder hauptsächlich daraus hergestellt ist, das vorzugsweise ausgewählt ist aus einer Gruppe, die besteht aus AcrylnitrilButadien-Styrol, Polymilchsäure und Nylon.

7. System nach einem der vorhergehenden Ansprüche, wobei die Stützwand eine durchschnittliche Dicke in einem Bereich von 1 bis 7 mm aufweist.

8. System nach dem vorhergehenden Anspruch, wobei die Stützwand eine durchschnittliche Dicke in einem Bereich von 3 bis 7 mm aufweist.

9. System nach einem der vorhergehenden Ansprüche, ferner umfassend ein Rechensystem (5) und Bildverarbeitungssoftware, die darin installiert ist und dazu ausgestaltet ist, eine 3-dimensionale Form des Stützmantels zu erzeugen und ferner eine Herstellungsdatei aus der 3D-Form des Stützmantels zur Übertragung an eine Stützmantel-Herstellungsmaschine zu erzeugen.

10. System nach einem der vorhergehenden Ansprüche, wobei das System ferner eine Stützmantel-Herstellungsmaschine (7) umfasst, die eine additive Fertigungsvorrichtung, zum Beispiel eine 3D-Druckvorrichtung, umfasst.

11. System nach einem der vorhergehenden Ansprüche, wobei das Führungsrohr zylindrisch ist.

12. System nach einem der vorhergehenden Ansprüche, wobei die Behandlungsvorrichtungspositioniereinrichtung und die Behandlungsvorrichtung wahlweise mit dem Vorrichtungsadapter (10) sich aneinander koppeln, wodurch eine spezifische Position auf dem Stützmantel und eine spezifische Zieltherapierichtung *(La)* in Bezug auf den Stützmantel definiert werden.

**13.** System nach dem vorhergehenden Anspruch, wobei die Behandlungsvorrichtungspositioniereinrichtung und die Behandlungsvorrichtung, wahlweise mit dem Vorrichtungsadapter (10), eine Grenzfläche mit einem vordefinierten Betrag an Spiel bilden, das einen angegebenen Freiheitsgrad in der Richtung der Behandlungsvorrichtung in Bezug auf die Behandlungsachse *(La)* zulässt, wobei die Anpassung innerhalb eines Kegels erfolgt, der einen Winkel von weniger als 10° aufweist.

**14.** System nach dem vorhergehenden Anspruch, wobei die Anpassung innerhalb eines Kegels erfolgt, der einen Winkel von weniger als 5° aufweist.

**Revendications**

**1.** Système d'irradiation avec traitement anticancéreux par hyperthermie (1) comprenant une coque de support rigide (2), la coque de support comprenant une paroi de conformation de corps (4) et au moins un localisateur de dispositif de traitement (6), le localisateur de dispositif de traitement comprenant un orifice (12) et un tube de guidage (14) se dressant vers l'extérieur à partir de la paroi de conformation de corps et étant configuré pour recevoir et localiser un dispositif de traitement (8) comprenant un applicateur d'hyperthermie à ultrasons (8a), lequel système comprend en outre un système de calcul de procédé de traitement (5') comprenant un module de programme configuré pour commander un procédé de traitement dans lequel des données d'une première session de HT sous la commande du MR sont enregistrées dans le système de calcul, le module de programme comprenant un module de calcul de fonction de Green configuré pour calculer une puissance au fil du temps prescrite, sur la base desdites données enregistrées d'une première session et d'une température de tissu cible, pour délivrer en sortie des données de commande de procédé pour commander l'applicateur d'hyperthermie à ultrasons dans des sessions subséquentes.

**2.** Système selon la revendication précédente, dans lequel la coque de support comprend au moins deux parties séparables qui peuvent être fixées ensemble de façon amovible pour un positionnement autour d'une partie du corps d'un patient.

**3.** Système selon la revendication précédente, comprenant en outre un adaptateur de dispositif (10) ayant une interface ajustable ou réglable se couplant au tube de guidage du localisateur de dispositif de traitement et une interface intérieure localisant le dispositif de traitement.

**4.** Système selon l'une quelconque des revendications précédentes, comprenant en outre ledit dispositif de traitement qui comprend un émetteur de rayonnement (8b).

**5.** Système selon l'une quelconque des revendications précédentes, comprenant en outre ledit dispositif de traitement qui comprend un dispositif de surveillance.

**6.** Système selon l'une quelconque des revendications précédentes, dans lequel la paroi de support consiste en ou est principalement faite en un matériau polymère, de préférence choisi dans le groupe constitué par l'acrylonitrile-butadiène-styrène, le poly(acide lactique) et le nylon.

**7.** Système selon l'une quelconque des revendications précédentes, dans lequel la paroi de support a une épaisseur moyenne située dans la plage allant de 1 à 7 mm.

**8.** Système la revendication précédente, dans lequel la paroi de support a une épaisseur moyenne située dans la plage allant de 3 à 7 mm.

**9.** Système selon l'une quelconque des revendications précédentes, comprenant en outre un système de calcul (5) et un logiciel de traitement d'image installés dans celui-ci et configurés pour générer une forme tridimensionnelle de la coque de support et en outre pour générer un fichier de fabrication à partir de la forme tridimensionnelle de la coque de support pour une transmission à une machine de fabrication de coque de support.

**10.** Système selon l'une quelconque des revendications précédentes, lequel système comprend en outre une machine de fabrication de coque de support (7) comprenant un dispositif de fabrication additive, par exemple un dispositif d'impression en trois dimensions.

**11.** Système selon l'une quelconque des revendications précédentes, dans lequel le tube de guidage est cylindrique.

**12.** Système selon l'une quelconque des revendications précédentes, dans lequel le localisateur de dispositif de traitement et le dispositif de traitement éventuellement avec l'adaptateur de dispositif (10) sont couplés ensemble en définissant une position spécifique sur la coque de support et une direction de thérapie ciblée spécifique *(La)* par rapport à la coque de support.

**13.** Système selon la revendication précédente, dans lequel le localisateur de dispositif de traitement et le dispositif de traitement, éventuellement avec l'adaptateur de dispositif (10), sont interfacés avec une quantité prédéfinie de jeu permettant un degré spécifique de liberté dans la direction du dispositif de traitement par rapport à l'axe de traitement *(La)*, l'ajustement étant à l'intérieur d'un cône ayant un angle inférieur à 10°.

**14.** Système selon la revendication précédente, dans lequel ledit ajustement est à l'intérieur d'un cône ayant un angle inférieur à 5°.

FIG. 1A

EP 4 486 439 B1

FIG. 1B

FIG. 2

**FIG. 3A**

**FIG. 3B**

EP 4 486 439 B1

FIG. 4

**FIG. 5**

Scanning system — 3

Computing system — 5

Software: (i) support shell shape generator
(ii) Machining instructions

Support Shell Manufacturing Machine — 7

Support Shell — 2

Treatment device — 8, 8a, 8b
- Hyperthermia Applicator
- Radiation emitter

Assembled Support shell & treatment device

**FIG. 6**

Position treatment device on patient → Scan body part → Compute support shell shape → Generate machining instructions → Manufacture Support Shell → Assemble Support shell & Treatment device on patient → Fine adjustment of treatment device → Perform Therapy

## FIG. 7

5'

**SESSION 1**

First session treatment inside MRI

First session treatment data

**Treatment Process Computing System**

Memory: recorded data

Process control instructions

Green Function Computation Program

ulterior treatment process control

SESSION 2

SESSION 3

SESSION 4

SESSION ...

Following treatment sessions performed in a basic examination room

FIG. 8

Excitation Power of HT
Ultrasound applicator

t

Measured tissue
Temperature

t

Green
function

Target tissue Temperature

t

Prescribed Power for HT
Ultrasound applicator

t

EP 4 486 439 B1

## REFERENCES CITED IN THE DESCRIPTION

**Patent documents cited in the description**

- US 2011040190 A

**Non-patent literature cited in the description**

- **CARRIE C** ; **MAGNE N** ; **BURBAN-PROVOST P et al.** Short-term androgen deprivation therapy combined with radiotherapy as salvage treatment after radical prostatectomy for prostate cancer (GETUG-AFU 16): a 112-month follow-up of a phase 3, randomised trial. *Lancet Oncol*, 2019, vol. 20 (12), 1740-1749
- **ACHARD V** ; **PANJE CM** ; **ENGELER D** ; **ZILLI T** ; **PUTORA PM**. Localized and Locally Advanced Prostate Cancer: Treatment Options. *Oncology*, 2021, 1-9
- **TENDULKAR RD** ; **AGRAWAL S** ; **GAO T et al.** Contemporary Update of a Multi-Institutional Predictive Nomogram for Salvage Radiotherapy After Radical Prostatectomy. *J Clin Oncol*, 2016, vol. 34 (30), 3648-3654
- **DAL PRA A** ; **PANJE C** ; **ZILLI T et al.** Salvage radiotherapy for macroscopic local recurrences after radical prostatectomy : A national survey on patterns of practice. *Strahlenther Onkol*, 2018, vol. 194 (1), 9-16
- **ZILLI T** ; **JORCANO S** ; **PEGURET N et al.** Results of Dose-adapted Salvage Radiotherapy After Radical Prostatectomy Based on an Endorectal MRI Target Definition Model. *Am J Clin Oncol*, 2017, vol. 40 (2), 194-199
- **ZILLI T** ; **JORCANO S** ; **PEGURET N et al.** Dose-adapted salvage radiotherapy after radical prostatectomy based on an erMRI target definition model: toxicity analysis. *Acta Oncol*, 2014, vol. 53 (1), 96-102
- **PICARDI C** ; **PERRET I** ; **MIRALBELL R** ; **ZILLI T**. Hypofractionated radiotherapy for prostate cancer in the postoperative setting: What is the evidence so far?. *Cancer Treat Rev*, 2018, vol. 62, 91-96
- **RICHTER A** ; **EXNER F** ; **WEICK S et al.** Evaluation of intrafraction prostate motion tracking using the Clarity Autoscan system for safety margin validation. *Z Med Phys*, 2020, vol. 30 (2), 135-141
- **DEWHIRST MW** ; **VUJASKOVIC Z** ; **JONES E** ; **THRALL D**. Re-setting the biologic rationale for thermal therapy. *Int J Hyperthermia*, 2005, vol. 21 (8), 779-790

- **DATTA NR** ; **PURIC E** ; **KLINGBIEL D** ; **GOMEZ S** ; **BODIS S**. Hyperthermia and Radiation Therapy in Locoregional Recurrent Breast Cancers: A Systematic Review and Meta-analysis. *Int J Radiat Oncol Biol Phys*, 2016, vol. 94 (5), 1073-1087
- **DATTA NR** ; **ROGERS S** ; **ORDONEZ SG** ; **PURIC E** ; **BODIS S**. Hyperthermia and radiotherapy in the management of head and neck cancers: A systematic review and meta-analysis. *Int J Hyperthermia*, 2016, vol. 32 (1), 31-40
- **DATTA NR** ; **STUTZ E** ; **GOMEZ S** ; **BODIS S**. Efficacy and Safety Evaluation of the Various Therapeutic Options in Locally Advanced Cervix Cancer: A Systematic Review and Network Meta-Analysis of Randomized Clinical Trials. *Int J Radiat Oncol Biol Phys*, 2019, vol. 103 (2), 411-437
- **CREZEE J** ; **VAN LEEUWEN CM** ; **OEI AL et al.** Biological modelling of the radiation dose escalation effect of regional hyperthermia in cervical cancer. *Radiat Oncol*, 2016, vol. 11, 14
- **BECK M** ; **GHADJAR P** ; **MEHRHOF F et al.** Salvage-Radiation Therapy and Regional Hyperthermia for Biochemically Recurrent Prostate Cancer after Radical Prostatectomy (Results of the Planned Interim Analysis). *Cancers (Basel)*, 2021, vol. 13 (5)
- **KOK HP** ; **CREZEE J** ; **FRANKEN NA** ; **STALPERS LJ** ; **BARENDSEN GW** ; **BEL A**. Quantifying the combined effect of radiation therapy and hyperthermia in terms of equivalent dose distributions. *Int J Radiat Oncol Biol Phys*, 2014, vol. 88 (3), 739-745
- **GUILLEMIN PC** ; **GUI L** ; **LORTON O et al.** Mild hyperthermia by MR-guided focused ultrasound in an ex vivo model of osteolytic bone tumour: optimization of the spatio-temporal control of the delivered temperature. *J Transl Med*, 2019, vol. 17 (1), 350
- **ZHU L** ; **LAM D** ; **PACIA CP et al.** Characterization of magnetic resonance-guided high-intensity focused ultrasound (MRgHIFU)-induced large-volume hyperthermia in deep and superficial targets in a porcine model. *Int J Hyperthermia*, 2020, vol. 37 (1), 1159-1173

- **BAUST JM** ; **RABIN Y** ; **POLASCIK TJ et al.** Defeating Cancers' Adaptive Defensive Strategies Using Thermal Therapies: Examining Cancer's Therapeutic Resistance, Ablative, and Computational Modeling Strategies as a means for Improving Therapeutic Outcome. *Technol Cancer Res Treat*, 2018, vol. 17, 1533033818762207
- **JOLESZ FA**. MRI-guided focused ultrasound surgery. *Annu Rev Med*, 2009, vol. 60, 417-430
- **MELODELIMA D** ; **SALOMIR R** ; **MOUGENOT C et al.** Intraluminal ultrasound applicator compatible with magnetic resonance imaging ''real-time'' temperature mapping for the treatment of oesophageal tumours: an ex vivo study. *Med Phys*, 2004, vol. 31 (2), 236-244
- **MOONEN CT** ; **QUESSON B** ; **SALOMIR R et al.** Thermal therapies in interventional MR imaging. Focused ultrasound. *Neuroimaging Clin N Am*, 2001, vol. 11 (4), 737-747
- **ZHOU YF**. High intensity focused ultrasound in clinical tumor ablation. *World J Clin Oncol*, 2011, vol. 2 (1), 8-27
- **MARIEN A** ; **GILL I** ; **UKIMURA O** ; **BETROUNI N** ; **VILLERS A**. Target ablation--image-guided therapy in prostate cancer. *Urol Oncol*, 2014, vol. 32 (6), 912-923
- **RUEFF LE** ; **RAMAN SS**. Clinical and Technical Aspects of MR-Guided High Intensity Focused Ultrasound for Treatment of Symptomatic Uterine Fibroids. *Semin Intervent Radiol*, 2013, vol. 30 (4), 347-353
- **ELLIS S** ; **RIEKE V** ; **KOHI M** ; **WESTPHALEN AC**. Clinical applications for magnetic resonance guided high intensity focused ultrasound (MRgHIFU): present and future. *J Med Imaging Radiat Oncol*, 2013, vol. 57 (4), 391-399
- **GUILHON E** ; **QUESSON B** ; **MORAUD-GAUDRY F et al.** Image-guided control of transgene expression based on local hyperthermia. *Mol Imaging*, 2003, vol. 2 (1), 11-17
- **ZHU L** ; **HUANG Y** ; **LAM D et al.** Targetability of cervical cancer by magnetic resonance-guided high-intensity focused ultrasound (MRgHIFU)-mediated hyperthermia (HT) for patients receiving radiation therapy. *Int J Hyperthermia*, 2021, vol. 38 (1), 498-510
- **ZHU L** ; **PARTANEN A** ; **TALCOTT MR et al.** Feasibility and safety assessment of magnetic resonance-guided high-intensity focused ultrasound (MRgHIFU)-mediated mild hyperthermia in pelvic targets evaluated using an in vivo porcine model. *Int J Hyperthermia*, 2019, vol. 36 (1), 1147-1159
- **GHADJAR P** ; **FIORINO C** ; **MUNCK AF ROSENSCHOLD P** ; **PINKAWA M** ; **ZILLI T** ; **VAN DER HEIDE UA**. ESTRO ACROP consensus guideline on the use of image guided radiation therapy for localized prostate cancer. *Radiother Oncol*, 2019, vol. 141, 5-13
- **MOSER T** ; **CREED M** ; **WALKER R** ; **MEIER G**. Radiotherapy tattoos: Women's skin as a carrier of personal memory-What do we cause by tattooing our patients?. *Breast J*, 2020, vol. 26 (2), 316-318
- **SUN J** ; **PICHLER P** ; **DOWLING J et al.** MR simulation for prostate radiation therapy: effect of coil mounting position on image quality. *Br J Radiol*, 2014, vol. 87 (1042), 20140325
- **FEDDERSEN TV** ; **HEMANDEZ-TAMAMES JA** ; **FRANCKENA M** ; **VAN RHOON GC** ; **PAULIDES MM**. Clinical Performance and Future Potential of Magnetic Resonance Thermometry in Hyperthermia. *Cancers (Basel)*, 2020, vol. 13 (1)
- **DIPASQUALE G** ; **POIRIER A** ; **SPRUNGER Y** ; **UITERWIJK JWE** ; **MIRALBELL R**. Improving 3D-printing of megavoltage X-rays radiotherapy bolus with surface-scanner. *Radiat Oncol*, 2018, vol. 13 (1), 203
- **FAHIMIAN B** ; **YU V** ; **HORST K** ; **XING L** ; **HRISTOV D**. Trajectory modulated prone breast irradiation: A LINAC-based technique combining intensity modulated delivery and motion of the couch. *Radiotherapy and Oncology*, 2013, vol. 109 (3), 475-481
- **SCHMIDHALTER D** ; **FIX MK** ; **WYSS M et al.** Evaluation of a new six degrees of freedom couch for radiation therapy. *Med Phys*, 2013, vol. 40 (11), 111710
- **SCHOFFEL PJ** ; **HARMS W** ; **SROKA-PEREZ G** ; **SCHLEGEL W** ; **KARGER CP**. Accuracy of a commercial optical 3D surface imaging system for realignment of patients for radiotherapy of the thorax. *Phys Med Biol*, 2007, vol. 52 (13), 3949-3963
- **BARTONCINI S** ; **FIANDRA C** ; **RUO REDDA MG** ; **ALLIS S** ; **MUNOZ F** ; **RICARDI U**. Target registration errors with surface imaging system in conformal radiotherapy for prostate cancer: study on 19 patients. *Radiol Med*, 2012, vol. 117 (8), 1419-1428
- **CHIESA S** ; **PLACIDI L** ; **AZARIO L et al.** Adaptive optimization by 6 DOF robotic couch in prostate volumetric IMRT treatment: rototranslational shift and dosimetric consequences. *J Appl Clin Med Phys*, 2015, vol. 16 (5)
- **LI W** ; **JIANG Z** ; **CHU K** ; **JIN J** ; **GE Y** ; **CAI J**. A Noninvasive Method to Reduce Radiotherapy Positioning Error Caused by Respiration for Patients With Abdominal or Pelvic Cancers. *Technol Cancer Res Treat*, 2019, vol. 18, 1533033819825865
- **MCPARTLIN AJ** ; **LI XA** ; **KERSHAW LE et al.** MRI-guided prostate adaptive radiotherapy - A systematic review. *Radiother Oncol*, 2016, vol. 119 (3), 371-380
- **GRIMWOOD A** ; **MCNAIR HA** ; **O'SHEA TP et al.** In Vivo Validation of Elekta's Clarity Autoscan for Ultrasound-based Intrafraction Motion Estimation of the Prostate During Radiation Therapy. *Int J Radiat Oncol Biol Phys*, 2018, vol. 102 (4), 912-921

- **WANG L** ; **CMELAK AJ** ; **DING GX**. A simple technique to improve calculated skin dose accuracy in a commercial treatment planning system. *J Appl Clin Med Phys*, 2018, vol. 19 (2), 191-197